# EUROPÄISCHE PATENTSCHRIFT

(11) **EP 0 975 570 B1**
(45) Veröffentlichungstag und Bekanntmachung des Hinweises auf die Patenterteilung: **04.09.2002**
(21) Anmeldenummer: 98924085.8
(22) Anmeldetag: 27.03.1998
(51) Int. Cl.: C07C 43/307, C11B 9/00, C07C 43/315

(54) **PHENONKETALE SOWIE DEREN VERWENDUNG ALS RIECHSTOFFE**
PHENOKETALS AND THE USE THEREOF AS ODORIFEROUS SUBSTANCES
CETALS DE PHENONE ET LEUR UTILISATION COMME SUBSTANCES ODORIFERANTES

(30) Priorität: 05.04.1997 DE 19714041
(43) Veröffentlichungstag der Anmeldung: 02.02.2000
(73) Patentinhaber: Cognis Deutschland GmbH & Co. KG, 40589 Düsseldorf (DE)
(72) Erfinder: MARKERT, Thomas, D-40789 Monheim (DE); NEMITZ, Ralph, D-41363 Jüchen (DE)
(86) Internationale Anmeldenummer: EP9801815
(87) Internationale Veröffentlichungsnummer: WO98045236

(56) Entgegenhaltungen:
- EP-A- 0 052 775
- US-A- 4 614 611
- KE-JUN CHENG ET AL: "A facile synthesis of 1,3,5-triarylbenzenes from acetophenone diethyl ketals in the presence of acetyl chloride and SmCl3" SYNTHETIC COMMUNICATIONS, Bd. 27, Nr. 1, 1997, Seiten 11-15, XP002073790

## Beschreibung

### Gebiet der Erfindung

Die Erfindung betrifft Phenonketale spezieller Struktur sowie deren Verwendung als Riechstoffe.

### Stand der Technik

Viele natürliche Riechstoffe stehen, gemessen am Bedarf, in völlig unzureichender Menge zur Verfügung. Beispielsweise sind zur Gewinnung von 1 kg Rosenöl 5.000 kg Rosenblüten notwendig; die Folgen sind eine sehr stark limitierte Weltjahresproduktion sowie ein hoher Preis. Es ist daher klar, daß die Riechstoffindustrie einen ständigen Bedarf an neuen Riechstoffen mit interessanten Duftnoten hat, um die Palette der natürlich verfügbaren Riechstoffe zu ergänzen und die notwendigen Anpassungen an wechselnde modische Geschmacksrichtungen vornehmen sowie den ständig steigenden Bedarf an Geruchsverbesserern für Produkte des täglichen Bedarfs wie Kosmetika und Reinigungsmittel decken zu können.

Es ist daher klar, daß die Riechstoffindustrie einen ständigen Bedarf an neuen Riechstoffen mit interessanten Duftnoten hat, um die Palette der natürlich verfügbaren Riechstoffe zu ergänzen und die notwendigen Anpassungen an wechselnde modische Geschmacksrichtungen vornehmen sowie den ständig steigenden Bedarf an Geruchsverbesserern für Produkte des täglichen Bedarfs wie Kosmetika und Reinigungsmittel decken zu können.

Darüber hinaus besteht generell ein ständiger Bedarf an synthetischen Riechstoffen, die sich günstig und mit gleichbleibender Qualität herstellen lassen und erwünschte olfaktorische Eigenschaften haben, d.h. angenehme, möglichst naturnahe und qualitativ neuartige Geruchsprofile von ausreichender Intensität besitzen und in der Lage sind, den Duft von kosmetischen und Verbrauchsgütern vorteilhaft zu beeinflussen. Mit anderen Worten: Es besteht ein ständiger Bedarf an Verbindungen, die charakteristische neue Geruchsprofile bei gleichzeitig hoher Haftfestigkeit, Geruchsintensität und Strahlkraft aufweisen.

US-A-4,614,611 beschreibt p-Methylacetophenon-dimethylacetal sowie dessen Verwendung als Riechstoff.

EP-A-052 775 beschreibt ein Verfahren zur Herstellung von aromatischen Aldehyden. Im Zuge dieses Verfahrens werden die entsprechenden Vorstufen einer Hydroformylierung unterworfen.

In Synthetic Communications 27 (1), 1997, S. 11-15, dienen spezielle Phenonketale als Edukte zur Herstellung von Triarylbenzolen.

### Beschreibung der Erfindung

Es wurde gefunden, daß spezielle Phenonketale der unten näher bezeichneten Struktur (I) die oben genannten Forderungen in jeder Hinsicht ausgezeichnet erfüllen und in vorteilhafter Weise als Riechstoffe mit unterschiedlich nuancierten Geruchsnoten mit guter Haftfestigkeit eingesetzt werden können.

Gegenstand der Erfindung ist die Verwendung von Phenonketalen der allgemeinen Struktur (I) 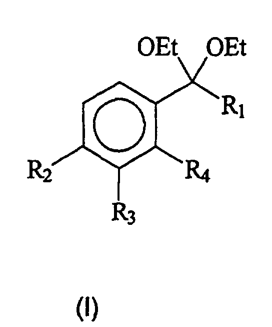 worin R₁ eine Methyl- oder Ethylgruppe, R₂ eine Methyl-, tert.-Butyl- oder Methoxygruppe, R₃ Wasserstoff oder eine Methoxygruppe und R₄ Wasserstoff oder eine Methylgruppe bedeuten, als Riechstoffe.

Bevorzugt ist insbesondere die Verbindung 1-(1,1-Diethoxyethyl)-2,4-dimethylbenzol, also das Diethylketal des 2,4-Dimethylacetophenons.

Die erfindungsgemäßen Carbonylverbindungen zeichnen sich durch eine Geruchscharakteristik aus , in der blumige, an Anthranilat, Ylang und Tuberose erinnernde Noten dominieren. Sie weisen eine ausgezeichnete Stabilität in Rezepturen der Kosmetik und Gebrauchsparfümerie auf.

Die Herstellung der Verbindungen (I) erfolgt nach an sich bekannten Syntheseverfahren der organischen Chemie. Die Synthese erfolgt beispielsweise durch Rühren der Ketone, die im gewünschten Alkohol gelöst sind und der im Überschuß eingesetzt wird, in Gegenwart wasserentziehender Mittel, die sowohl in katalytischen Mengen (0,01 bis 0,2 Mol- %) als auch in molaren Mengen (beim Einsatz von Molsieben) - jeweils bezogen auf die Ketone - eingesetzt werden können. Als wasserentziehende Mittel eignen sich beispielsweise Sulfate wie Magnesium-, Natrium- oder Kaliumsulfate bzw. - hydrogensulfate oder Sulfonsäuren wie beispielsweise p-Toluolsulfonsäure, Pyridiniumtosylat etc. sowie Molsiebe (vorzugsweise mit einem Porendurchmesser von ca. 4 Angström).

Die erfindungsgemäßen Ketale sind dabei durch Abdestillieren des überschüssigen Alkohols und Fraktionierung des Rohproduktes in sehr reiner Form zugänglich.

In Parfüm-Kompositionen verstärken die Verbindungen (I) die Harmonie und Ausstrahlung und Natürlichkeit sowie auch die Haftung, wobei die Dosierung unter Berücksichtigung der übrigen Bestandteile der Komposition auf die jeweils angestrebte Duftnote abgestimmt wird.

Daß die Carbonylverbindungen (I) blumige, Anthranilat-, Ylang- und Tuberose-Noten aufweisen, war nicht vorhersehbar und ist damit eine weitere Bestätigung für die allgemeine Erfahrung, daß die olfaktorischen Eigenschaften bekannter Riechstoffe keine zwingenden Rückschlüsse auf die Eigenschaften strukturverwandter Verbindungen zulassen, weil weder der Mechanismus der Duftwahrnehmung noch der Einfluß der chemischen Struktur auf die Duftwahrnehmung hinreichend erforscht sind, somit also normalerweise nicht vorhergesehen werden kann, ob ein geänderter Aufbau bekannter Riechstoffe überhaupt zur Änderung der olfaktorischen Eigenschaften führt und ob diese Änderungen positiv oder negativ beurteilt werden.

Die Verbindungen der Formel (I) eignen sich aufgrund ihres Geruchsprofils insbesondere auch zur Modifizierung und Verstärkung bekannter Kompositionen. Hervorgehoben werden soll insbesondere ihre außerordentliche Geruchsstärke, die ganz allgemein zur Veredelung der Komposition beiträgt.

Die Verbindungen der Formel (I) lassen sich mit zahlreichen bekannten Riechstoffingredientien, z.B. anderen Riechstoffen natürlichen, synthetischen oder partialsynthetischen Ursprungs, etherischen Ölen und Pflanzenextrakten kombinieren. Die Palette der natürlichen Riechstoffe kann dabei sowohl leicht- als auch mittelund schwerflüchtige Komponenten und diejenige der synthetischen Riechstoffe Vertreter aus praktisch allen Stoffklassen umfassen. Beispiele sind:
(a) Naturprodukte wie Baummoos-Absolue, Basilikumöl, Agrumenöle wie Bergamotteöl, Mandarinenöl, usw., Mastix-Absolue, Myrtenöl, Palmarosaöl, Patchouliöl, Petitgrainöl, Wermutöl,Myrrheöl, Olibanumöl
(b) Alkohole wie Farnesol, Geraniol, Linalool, Nerol, Phenylethylalkohol, Rhodinol, Zimtalkohol, Sandalore [3-Methyl-5-(2.2.3-trimethylcyclopent-3-en-1-yl)pentan-2-ol], Sandela [3-Isocamphyl-(5)-cyclohexanol],
(c) Aldehyde wie Citral, Helional^{R}, µ-Hexylzimtaldehyd, Hydroxycitronellal, Lilial^{R} [p-tert.-Butyl-µ-methyldihydrozimtaldehyd], Methylnonylacetaldehyd,
(d) Ketone wie Allylionon, µ-Ionon, β-Ionon, Isoraldein, Methylionon,
(e) Ester wie Allylphenoxyacetat, Benzylsalicylat, Cinnamylpropionat, Citronellylacetat, Decylacetat, Dimethylbenzylcarbinylacetat, Ethylacetoacetat, Hexenylisobutyrat, Linalylacetat, Methyldihydrojasmonat, Vetiverylacetat, Cyclohexylsalicylat,
(f) Lactone wie gamma-Undecalacton, 1-Oxaspiro[4.4]nonan-2-on,
sowie verschiedene weitere in der Parfümerie oft benutzte Komponenten wie Moschus- und Sandelholz-Riechstoffe, Indol, p-Menthan-8-thiol-3-on, Methyleugenol, Ambroxan.

Bemerkenswert ist ferner die Art und Weise, wie die Verbindungen der Struktur (I) die Geruchsnoten einer breiten Palette bekannter Kompositionen abrunden und harmonisieren, ohne aber in unangenehmer Weise zu dominieren. 1-(1,1-Dioxyethyl)-2,4-dimethylbenzol, also das Diethylketal des 2,4-Dimethylacetophenons, ist in dieser Hinsicht ganz besonders hervorzuheben.

Die einsetzbaren Anteile der erfindungsgemäßen Verbindungen oder deren Gemische in Riechstoffkompositionen bewegen sich von 1 bis 70 Gewichtsprozent, bezogen auf die gesamte Mischung. Gemische der erfindungsgemäßen Verbindungen (I) sowie Kompositionen dieser Art können sowohl zur Parfümierung kosmetischer Präparate wie Lotionen, Cremes, Shampoos, Seifen, Salben, Puder, Aerosole, Zahnpasten, Mundwässer, Deodorantien als auch in der alkoholischen Parfümerie (z.B. Eaux de Cologne, Eaux de Toilette, Extraits) verwendet werden. Ebenso besteht eine Einsatzmöglichkeit zur Parfümierung technischer Produkte wie Waschund Reinigungsmittel, Weichspüler und Textilbehandlungsmittel. Zur Parfümierung dieser verschiedenen Produkte werden diesen die Kompositionen in einer olfaktorisch wirksamen Menge, insbesondere in einer Konzentration von 0,05 bis 2 Gewichtsprozent, bezogen auf das gesamte Produkt, zugesetzt. Diese Werte sollen jedoch keine Grenzwerte darstellen, da der erfahrene Parfümeur auch mit noch geringeren Konzentrationen Effekte erzielen oder aber mit noch höheren Dosierungen neuartige Komplexe aufbauen kann.

Die folgenden Beispiele dienen der Erläuterung der Erfindung und sind nicht einschränkend zu verstehen.

### Beispiele

### Beispiel 1: 1-(1,1-Diethoxyethyl)-2,4-dimethylbenzol

### Ansatz:

1) 97,3 g (0,66 Mol) 2,4-Dimethylacetophenon (Fa. Fluka)
2) 117,3 g (0,79 Mol) Triethylorthoformiat (Fa. Fluka)
3) 123,7 mg Kaliumhydrogensulfat (Fa. Merck)
4) 600 ml Ethanol, wasserfrei (technische Ware)

### Ausführung:

Unter Feuchtigkeitsausschluß (mit Kieselgel gefülltes Trockenrohr) wurden die Substanzen 1) bis 4) bei 20 °C unter Durchleiten von ca 2 l/h trockenem Stickstoff in einem 1-Dreihalskolben über 78 Stunden gerührt. Danach war über GLC kein Edukt mehr im Gemisch nachweisbar. Der Katalysator wurde zur Aufarbeitung mit 2 ml Natriummethylatlösung (30 % in Methanol) neutralisiert und die Mischung 15 Minuten gerührt. Danach wurde das Ethanol am Rotationsverdampfer abgezogen und die verbleibenden 150,9 g hellbrauner Rückstand zur Destillation an einer 15 cm Füllkörper-Kolonne (Braunschweiger Wendeln) eingesetzt. Es wurden 133,5 g Hauptlauf mit einem Siedepunkt von 73 - 75 °C/0,1 mbar erhalten. Die gaschromatographisch bestimmte Reinheit betrug 94,6 %.

**Geruchsbeschreibung:** Moschus-, Anthranilat-, Schwefelkautschuk-Note

### Beispiel 2: (1,1-Diethoxybutyl)-benzol

### Ansatz:

1) 148,0 h (1,0 Mol) Phenylpropylketon (Fa. Fluka)
2) 177,8 g (1,2 Mol) Triethylorthoformiat (Fa. Fluka)
3) 187,4 mg Kaliumhydrogensulfat (Fa. Merck)
4) 600 ml Ethanol, wasserfrei (technische Ware)

### Ausführung:

Unter Feuchtigkeitsausschluß (mit Kieselgel gefülltes Trockenrohr) wurden die Substanzen 1) bis 4) bei 20 °C unter Durchleiten von ca. 2 l/h trockenem Stickstoff in einem 1 1-Dreihalskolben über 78 Stunden gerührt. Danach waren über GLC nur noch 6 % Edukt im Gemisch nachweisbar. Der Katalysator wurde zur Aufarbeitung mit 2 ml Natriummethylatlösung (30 % in Methanol) neutralisiert und die Mischung 15 Minuten gerührt. Danach wurde das Ethanol am Rotationsverdampfer abgezogen und die verbleibenden 195,4 g hellorange Flüssigkeit zur Destillation an einer 15 cm Füllkörper-Kolonne (Braunschweiger Wendeln) eingesetzt. 182,9 g Hautlauf mit einem Siedepunkt von 74 - 78 °C/0,1 mbar wurden isoliert. Die gaschromatographisch bestimmte Reinheit betrug 88,9 %.
**Geruchsbeschreibung:** frisch, blumig, EdT-, Citrus-Note

### Beispiel 3: 1-(1,1-Diethoxyethyl)-3,4-dimethoxybenzol

### Ansatz:

1) 180,0 g (1,0 Mol) 3,4-Dimethoxyacetophenon (Fa. Merck)
2) 177,8 g (1,2 Mol) Triethylorthoformiat (Fa. Fluka)
3) 187,4 mg Kaliumhydrogensulfat (Fa. Merck)
4) 600 ml Ethanol, wasserfrei (technische Ware)

### Ausführung:

Unter Feuchtigkeitsausschluß (mit Kieselgel gefülltes Trockenrohr) wurden die Substanzen 1) bis 4) bei 20 °C unter Durchleiten von ca. 2 l/h trockenem Stickstoff in einem 1 1-Dreihalskolben ca. 20 Stunden gerührt. Danach waren über GLC nur noch 10 % Edukt im Gemisch nachweisbar. Der Katalysator wurde zur Aufarbeitung mit 2 ml Natriummethylatlösung (30 % in Methanol) neutralisiert und die Mischung 15 Minuten gerührt. Danach wurde das Ethanol am Rotationsverdampfer abgezogen und die verbleibenden 254,0 g braune Flüssigkeit zur Destillation an einer 15 cm Füllkörper-Kolonne (Braunschweiger Wendeln) eingesetzt. 197,3 g Hautlauf mit einem Siedepunkt von 102 - 103 °C/0,1 mbar wurden isoliert. Die gaschromatographisch bestimmte Reinheit betrug 99,7 %.
**Geruchsbeschreibung:** frisch, blumig, Dihydrojasmonat-Note

### Beispiel 4: 1-(1,1-Diethoxyethyl)-4-ethylbenzol

### Ansatz:

1) 148,0 g (1,0 Mol) 4-Ethylacetophenon (Fa. Aldrich)
2) 177,7 g (1,2 Mol) Triethylorthoformiat (Fa. Fluka)
3) 187,4 mg Kaliumhydrogensulfat (Fa. Merck)
4) 600 ml Ethanol, wasserfrei (technische Ware)

### Ausführung:

Unter Feuchtigkeitsausschluß (mit Kieselgel gefülltes Trockenrohr) wurden die Substanzen 1) bis 4) bei 20 °C unter Durchleiten von ca. 2 l/h trockenem Stickstoff in einem 1 1-Dreihalskolben über 20 Stunden gerührt. Danach war über GLC nur noch Produkt im Gemisch nachweisbar. Der Katalysator wurde zur Aufarbeitung mit 2 ml Natriummethylatlösung (30 % in Methanol) neutralisiert und die Mischung 15 Minuten gerührt. Danach wurde das Ethanol am Rotationsverdampfer abgezogen und die verbleibenden 228,3 g braune Flüssigkeit zur Destillation an einer 15 cm Füllkörper-Kolonne (Braunschweiger Wendeln) eingesetzt. 201,2 g Hautlauf mit einem Siedepunkt von 67- 70 °C/0,1 mbar wurden isoliert. Die gaschromatographisch bestimmte Reinheit betrug 98,7 %.
**Geruchsbeschreibung:** fruchtig, wie p-tert.-Butyl-cyclohexylacetat, 25 % cis

### Beispiel 5: 1-(1,1-Diethoxypropyl)-4-methylbenzol

### Ansatz:

1) 99,0 g (0,67 Mol) 4-Hethylpropiophenon (Fa. Acros)
2) 121,5 g (0,82 Mol) Triethylorthoformiat (Fa. Fluka)
3) 127,4 mg Kaliumhydrogensulfat (Fa. Merck)
4) 600 ml Ethanol, wasserfrei (technische Ware)

### Ausführung:

Unter Feuchtigkeitsausschluß (mit Kieselgel gefülltes Trockenrohr) wurden die Substanzen 1) bis 4) bei 20 °C unter Durchleiten von ca. 2 l/h trockenem Stickstoff in einem 1 1-Dreihalskolben über 20 Stunden gerührt. Danach war über GLC nur noch 10 % Edukt im Gemisch nachweisbar. Der Katalysator wurde zur Aufarbeitung mit 2 ml Natriummethylatlösung (30 % in Methanol) neutralisiert und die Mischung 15 Minuten gerührt. Danach wurde das Ethanol am Rotationsverdampfer abgezogen und die verbleibenden 156,0 g braune Flüssigkeit zur Destillation an einer 15 cm Füllkörper-Kolonne (Braunschweiger Wendeln) eingesetzt. 110,6 g Hautlauf mit einem Siedepunkt von 62 - 63 °C/0,1 mbar wurden isoliert. Die gaschromatographisch bestimmte Reinheit betrug 93,2 %.
**Geruchsbeschreibung:** Kümmel-, Tuberose, Ylang-Note, fruchtig

### Beispiel 6: 4-tert.-Butyl-1-(1,1-diethoxyethyl)benzol

### Ansatz:

1) 49,8 g (0,28 Mol) 4-tert.-Butylacetophenon (Fa. Interchim)
2) 50,0 g (0,34 Mol) Triethylorthoformiat (Fa. Fluka
3) 52,5 mg Kaliumhydrogensulfat (Fa. Merck)
4) 300 ml Ethanol, wasserfrei (technische Ware)

### Ausführung:

Unter Feuchtigkeitsausschluß (mit Kieselgel gefülltes Trockenrohr) wurden die Substanzen 1) bis 4) bei 20 °C unter Durchleiten von ca. 2 l/h trockenem Stickstoff in einem 1 1-Dreihalskolben über 20 Stunden gerührt. Danach zeigte das GLC ca. 92 % Produkt im Gemisch an. Der Katalysator wurde zur Aufarbeitung mit 2 ml Natriummethylatlösung (30 % in Methanol) neutralisiert und die Mischung 15 Minuten gerührt. Danach wurde das Ethanol am Rotationsverdampfer abgezogen und die verbleibenden 75,1 g braune Flüssigkeit zur Destillation an einer 30 cm Füllkörper-Kolonne (Braunschweiger Wendeln) eingesetzt. 110,6 g Hautlauf mit einem Siedepunkt von 72 - 76 °C/0,1 mbar wurden isoliert. Die gaschromatographisch bestimmte Reinheit betrug 98,1 %.
**Geruchsbeschreibung:** etwas fruchtig, blumig, holzig

## Patentansprüche

1. Verwendung von Phenonketalen der allgemeinen Struktur (I) 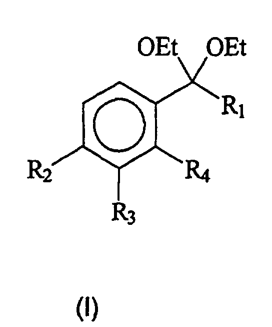 worin R₁ eine Methyl- oder Ethylgruppe, R₂ eine Methyl-, tert.-Butyl- oder Methoxygruppe, R₃ Wasserstoff oder eine Methoxygruppe und R₄ Wasserstoff oder eine Methylgruppe bedeuten, als Riechstoffe.

2. Verwendung von 1-(1,1-Diethoxyethyl)-2,4-dimethylbenzol als Riechstoff.

3. Riechstoffkompositionen mit einem Gehalt an einem oder mehreren Phenonketalen der allgemeinen Struktur (I) 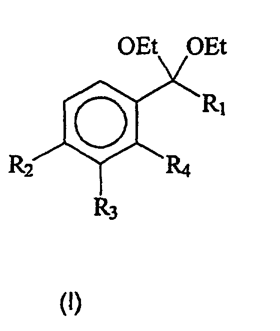 worin R₁ eine Methyl- oder Ethylgruppe, R₂ eine Methyl-, tert.-Butyl- oder Methoxygruppe, R₃ Wasserstoff oder eine Methoxygruppe und R₄ Wasserstoff oder eine Methylgruppe bedeuten, in einer Menge von 1 bis 70 Gew.-% (bezogen auf die gesamte Komposition).

4. 1-(1,1-Diethoxyethyl)-2,4-dimethylbenzol.

5. (1,1-Diethoxybutyl)-benzol.

6. 1-(1,1-Diethoxyethyl)-3,4-dimethoxybenzol.

7. 1-(1,1-Diethoxyethyl)-4-ethylbenzol.

8. 1-(1,1-Diethoxypropyl)-4-methylbenzol.

## Claims

1. The use of phenone ketals corresponding to general formula (I): 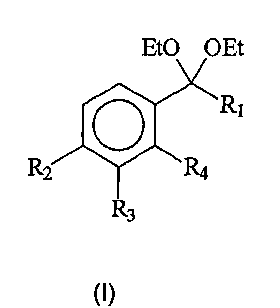 where R₁ is a methyl or ethyl group, R₂ is a methyl, tert.butyl or methoxy group, R₃ is hydrogen or a methoxy group and R₄ is hydrogen or a methyl group,
as perfumes.

2. The use of 1-(1,1-diethoxyethyl)-2,4-dimethyl benzene as a perfume.

3. Perfume compositions containing one or more phenone ketals corresponding to general formula (I): 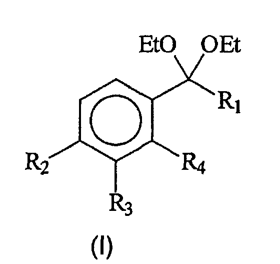 where R₁ is a methyl or ethyl group, R₂ is a methyl, tert.butyl or methoxy group, R₃ is hydrogen or a methoxy group and R₄ is hydrogen or a methyl group,
in a quantity of 1 to 70% by weight (based on the composition as a whole).

4. 1-(1,1-diethoxyethyl)-2,4-dimethyl benzene

5. (1,1-diethoxybutyl)-benzene.

6. 1-(1,1-diethoxyethyl)-3,4-dimethoxybenzene

7. 1-(1,1-diethoxyethyl)-4-ethyl benzene

8. 1-(1,1-diethoxypropyl)-4-methyl benzene.

## Revendications

1. Utilisation de cétals de phénone de structure générale (I): 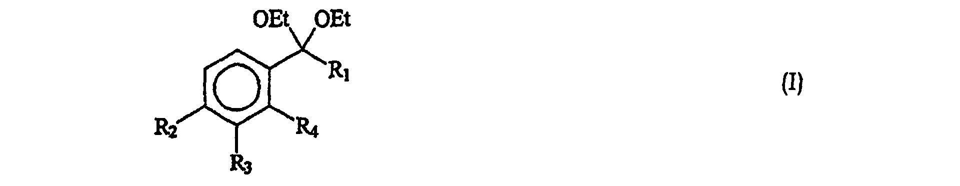 dans laquelle
R₁ signifie un groupe méthyle ou éthyle,
R₂ signifie un groupe méthyle, terbutyle ou méthoxy,
R₃ signifie de l'hydrogène ou un groupe méthoxy, et
R₄ signifie de l'hydrogène ou un groupe méthyle,
en tant que substances odorantes.

2. Utilisation du 1-(1,1-diéthoxyéthyl)-2,4-diméthylbenzène en tant que substance odorante.

3. Compositions de substances odorantes ayant une teneur en un ou plusieurs cétals de phénone de structure générale (I), 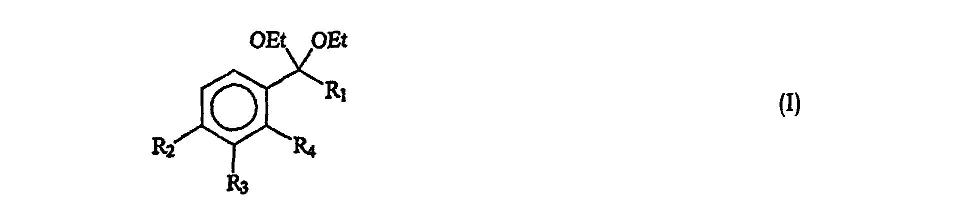 dans laquelle
R₁ signifie un groupe méthyle ou éthyle,
R₂ signifie un groupe méthyle, terbutyle ou méthoxy,
R₃ signifie de l'hydrogène ou un groupe méthoxy, et
R₄ signifie de l'hydrogène ou un groupe méthyle,
en une quantité allant de 1 à 70 % en poids (rapporté à la composition totale).

4. 1-(1,1-diéthoxyéthyl)-2,4-diméthylbenzène.

5. (1,1-diéthoxybutyl)-benzène.

6. 1-(1,1-diéthoxyéthyl)-3,4-diméthoxybenzène.

7. 1-(1,1-diéthoxyéthyl)-4-éthylbenzène.

8. 1-(1,1-diéthoxyéthyl)-4-méthylbenzène.
